# EUROPEAN PATENT SPECIFICATION

(11) **EP 3 168 602 B1**
(45) Date of publication and mention of the grant of the patent: **02.10.2019**
(21) Application number: 15818332.7
(22) Date of filing: 24.06.2015
(51) Int. Cl.: G01N 21/359, G01N 33/02, G01J 3/02, G01J 3/18, G01J 3/36, G01J 3/42, G01N 21/31, G01N 21/65, G01J 3/28, G01J 3/10

(54) **FOOD PRODUCT ANALYSIS DEVICE**
LEBENSMITTELPRODUKTANALYSEVORRICHTUNG
DISPOSITIF D'ANALYSE DE PRODUITS ALIMENTAIRES

(30) Priority: 11.07.2014 JP 2014143039
(43) Date of publication of application: 17.05.2017
(73) Proprietor: Panasonic Intellectual Property Management Co., Ltd., Osaka-shi, Osaka 570-6207 (JP)
(72) Inventor: OCHI, Kazuhiro, Osaka-shi, Osaka 540-6207 (JP)
(74) Representative: Grünecker Patent- und Rechtsanwälte PartG mbB
(86) International application number: PCT/JP2015/003165
(87) International publication number: WO 2016/006184

(56) References cited:
- WO-A1-2006/046197
- JP-A- 2006 226 945
- JP-A- 2006 333 815
- JP-A- 2007 047 930
- JP-A- 2008 122 412
- JP-A- 2013 036 905
- JP-A- 2013 036 906
- JP-A- 2013 040 832
- JP-A- 2014 062 807
- US-A1- 2012 206 731
- US-A1- 2014 185 046

## Description

### TECHNICAL FIELD

The present invention relates to a food product analysis device.

### BACKGROUND ART

A food product analysis device which analyzes the types of food products is known. In PTL 1, an example of the food product analysis device is disclosed. The types of the food products can be analyzed from image data obtained by imaging the food products.

Meanwhile, a dish, or the like for placing the food products may be reflected in the image data obtained by imaging the food products. In this case, the food product analysis device of PTL 1 may recognize a pattern formed on the dish, or the like, as the food product. Therefore, there is a concern that the types of the food products are not accurately analyzed.

### Citation List

### Patent Literature

PTL 1: Japanese Patent Unexamined Publication No. 2007-122311
PTL 2: WO 2006/046192 A1 relates to a device for determining the calorie content of food object. For a user-friendly determination of calorie contents a technique is provided, which easily and automatically determinates calorie values is by combination of different examination results. The examination result is obtained in a completely automatic way. A detailed user interaction, e.g. by pressing certain keys etc., is not necessary.
PTL 3: US 2014/185046 A1 relates to a food analysis device with a variable wavelength filter adapted to disperse light reflected by the food into a plurality of lights with respective wavelengths, an imaging section adapted to image the lights with the respective wavelengths obtained by the dispersion to obtain spectral images corresponding respectively to the wavelengths, and a control section adapted to obtain spectrum of each of the pixels from the spectral images corresponding to the respective wavelengths, and then detect a pixel including the absorption spectrum of water, and then detect a plurality of components based on the spectrum of the pixel detect.
PTL 4: JP 2008 122412 relates to a device for measuring the calories of food items.
PTL 5: US 2012/206731 A1 describes a system for detecting the presence of specific substances.
PTL 6: JP 2013 036905 A relates to a food product calories estimating process. Separate images of each wavelength range are captured with and without irradiating light from a light source to an imaging target. Subsequently, while irradiating light from the light source to the imaging target, images of each wavelength range are captured. Subsequently, from the captured image, a portion (i.e. the volume) of the food is extracted using the visible light image.

### SUMMARY OF THE INVENTION

The scope of the present invention is defined by the appended claims. In particular, the invention is directed to a food product analysis device as defined in claim 1.

The food product analysis device according to the invention is capable of accurately analyzing the types of food products.

### BRIEF DESCRIPTION OF DRAWINGS

FIG. 1 is a schematic view of food product analysis device according to Embodiment 1 of the invention.
FIG. 2 is a schematic view illustrating a configuration of a light receiver of the food product analysis device according to Embodiment 1 of the invention.
FIG. 3 is a block diagram of an analysis unit of the food product analysis device according to Embodiment 1 of the invention.
FIG. 4 is a front view illustrating a display example of a list of candidates which is displayed on a display portion of the food product analysis device according to Embodiment 1 of the invention.
FIG. 5 is a front view illustrating a display example of an analysis result which is displayed on the display portion of the food product analysis device according to Embodiment 1 of the invention.
FIG. 6 is a schematic view illustrating a configuration of a light receiver of food product analysis device according to Embodiment 2 of the invention.
FIG. 7 is a block diagram of an analysis unit of the food product analysis device according to Embodiment 2 of the invention.
FIG. 8 is a front view illustrating a display example of an overlapped image relating to calories which is displayed on the display portion of the food product analysis device according to Embodiment 2 of the invention.
FIG. 9 is a front view illustrating a display example of image data of food products which is displayed on a display portion of a food product analysis device according to a modification example of the embodiment of the invention.
FIG. 10 is a schematic view of the food product analysis device according to the modification example of the embodiment of the invention.
FIG. 11 is a schematic view of the food product analysis device according to the modification example of the embodiment of the invention.

### DESCRIPTION OF EMBODIMENTS

### (EMBODIMENT 1)

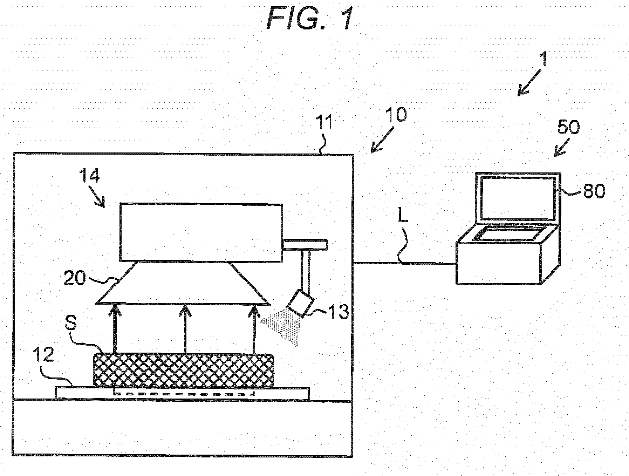

FIG. 1 is a schematic view of food product analysis device 1 according to Embodiment 1 of the invention.

Referring to FIG. 1, a configuration of food product analysis device 1 will be described.

Food product analysis device 1 is provided with measuring unit 10 measuring information relating to target S which is a food product, and analysis unit 50 analyzing a type, or the like of target S. Measuring unit 10 and analysis unit 50 are electrically connected to each other through connection cable L.

Measuring unit 10 is provided with housing 11 which is capable of light-shielding the inside by closing a door (not illustrated). Housing 11 accommodates table 12 in which is disposed target S placed on a dish, which is a target to be measured, irradiator 13 which applies near infrared light to target S, and light receiver 14 which receives light reflected from target S.

Irradiator 13 is a halogen lamp, a light emitting diode (LED), or a laser, as an example, and is disposed on a position capable of applying light to the entirety of target S. Irradiator 13 applies, as an example, light including a wavelength of an infrared region of 700 nm to 2500 nm.

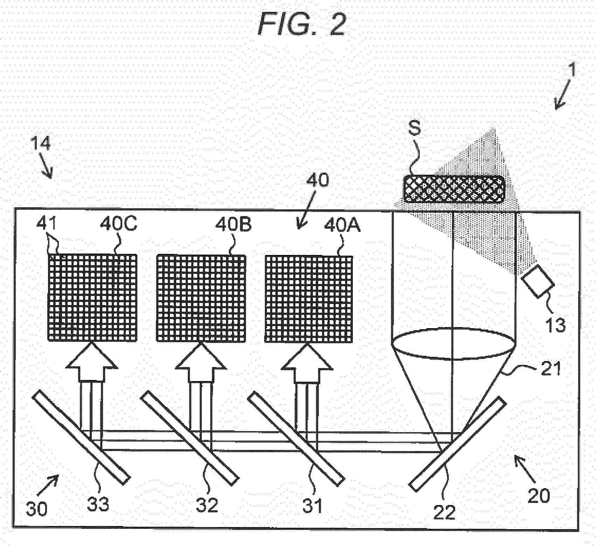

FIG. 2 is a schematic view illustrating a configuration of light receiver 14 of food product analysis device 1 according to Embodiment 1 of the invention.

Refer to FIG. 2, the configuration of light receiver 14 will be described.

Light receiver 14 is disposed on a position capable of receiving light which is reflected from target S. Light receiver 14 is provided with light condenser 20 which condenses the light reflected from target S, light dispersing portion 30 which disperses light of light of an absorption wavelength relating to ingredients of target S from the condensed light, and a plurality of light receiving sensors 40 which receives the dispersed light.

Light condenser 20 is provided with light condensing lens 21 for condensing light, and reflector 22 which guides the condensed light by light condensing lens 21 to light dispersing portion 30.

Light dispersing portion 30 is provided with first light dispersing portion 31, second light dispersing portion 32, and third light dispersing portion 33. Each of light dispersing portions 31 to 33 is a spectroscope of a diffraction grating which reflects only light of a specific wavelength and transmits light of a different wavelength. Light dispersing portions 31 to 33 only reflects light of a different specific wavelength from each other. These specific wavelengths are determined by experiments, and the like based on absorbance of a plurality of samples relating to the food products, in which ingredients thereof are known. Specifically, these specific wavelengths are determined as, for example, wavelengths, to which an ingredient amount of a specific ingredient of in samples is reflected, from a relationship between the ingredient amount of the specific ingredient and absorbance in a plurality of the samples.

First light dispersing portion 31 selectively reflects light of wavelength of around 910 nm correlating with an ingredient amount of protein among ingredients of the food product. Second light dispersing portion 32 selectively reflects light of wavelength of around 930 nm correlating with an ingredient amount of lipid among ingredients of the food product. Third light dispersing portion 33 selectively reflects light of wavelength of around 980 nm correlating with an ingredient amount of carbohydrate among ingredients of the food product.

An example of light receiving sensor 40 is an image sensor. The plurality of light receiving sensors 40 includes first light receiving sensor 40A, second light receiving sensor 40B, and third light receiving sensor 40C. First light receiving sensor 40A receives the reflected light in first light dispersing portion 31. Second light receiving sensor 40B receives the reflected light in second light dispersing portion 32. Third light receiving sensor 40C receives the reflected light in third light dispersing portion 33.

A plurality of light receiving elements 41 is disposed in a grid shape on a light receiving surface of each of light receiving sensors 40A to 40C. As a material of light receiving element 41, as an example, silicon, indium, gallium, arsenic, or the like is used, which has sensitivities in a wide range in the infrared region. Each light receiving elements 41 detects an amount of the received light.

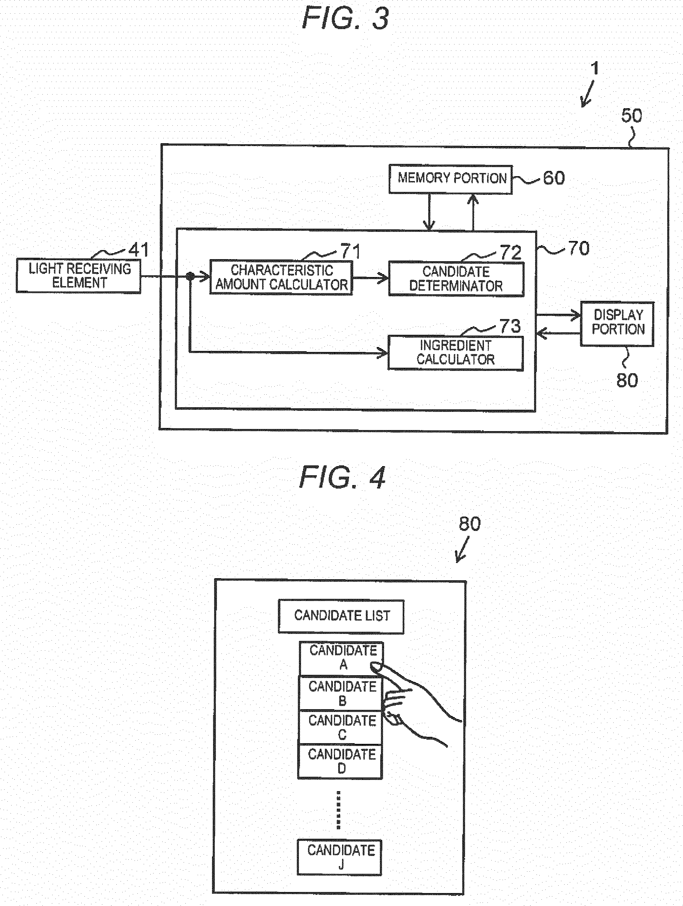

FIG. 3 is a block diagram of analysis unit 50 of food product analysis device 1 according to Embodiment 1 of the invention.

Referring to FIG. 3, a configuration of analysis unit 50 will be described.

Analysis unit 50 is provided with memory portion 60 which stores information, or the like relating to the samples, analysis portion 70 which analyses types, and the like of target S (refer to FIG. 1), and display portion 80 which displays an analysis result of analysis portion 70.

Memory portion 60 stores food product database containing multiple food product information. The food product information includes characteristic information including first light absorption information, which relates to absorption of near infrared light, and type information which relates to the types of samples. In addition, memory portion 60 stores an ingredient amount estimating model indicating a correlation between absorbance of samples which are calculated on the basis of a light receiving amount detected by light receiving element 41 and the ingredient amounts of the samples. The ingredient amount estimating model is calculated using a statistical method such as a multiple regression analysis or a partial least squares (PLS) regression analysis. In addition, the ingredient amount of the sample is an ingredient amount of, for example, protein, lipid, and carbohydrate, and is accurately calculated by another analysis method in the related art. Memory portion 60 stores information relating to an analysis result analyzed by analysis portion 70 at any time.

Analysis portion 70 is provided with characteristic amount calculator 71 which calculates a characteristic amount of target S, candidate determinator 72 which matches the calculated characteristic amount and the food product database and determines a plurality of results by matching, and an ingredient calculator 73 which calculates ingredients of target S.

Characteristic amount calculator 71 calculates an average value M of the absorbance of target S as a first characteristic amount relating to target S which is measured information. The average value M of the absorbance of target S is calculated on the basis of a light receiving amount, which is detected by a plurality of light receiving elements 41 disposed on first light receiving sensor 40A (refer to FIG. 2). Characteristic amount calculator 71 calculates average value Q of the absorbance of target S as a second characteristic amount relating to target S which is measured information. Average value Q of the absorbance of target S is calculated on the basis of the light receiving amount, which is detected by the plurality of light receiving elements 41 disposed on second light receiving sensor 40B (refer to FIG. 2). Characteristic amount calculator 71 calculates average value R of the absorbance of target S as a third characteristic amount relating to target S which is the measured information. Average value R of the absorbance of target S is calculated on the basis of the light receiving amount, which is detected by the plurality of light receiving elements 41 disposed on third light receiving sensor 40C (refer to FIG. 2). The characteristic information in the food product database is the first characteristic amount to the third characteristic amount relating to the samples.

Candidate determinator 72 matches, for example, the first characteristic amount to the third characteristic amount relating to target S with the first characteristic amount to the third characteristic amount relating to the samples in the food product database, and determines a plurality of results by the matching. Specifically, for example, a difference between the first characteristic amount relating to target S and the first characteristic amount relating to the sample, a difference between the second characteristic amount relating to target S and the second characteristic amount relating to the sample, and a difference between the third characteristic amount relating to target S and the third characteristic amount relating to the sample are calculated. Next, a matched result which is an average of each calculated difference is calculated. N candidates of the food product are determined from type information in order of small matched result. An example of N is ten. Candidate determinator 72 outputs information relating to the candidates to display portion 80.

Ingredient calculator 73 outputs the ingredient amount of the ingredients of target S based on the absorbance and the ingredient amount estimating model of target S. Ingredient calculator 73 calculates, as an example, an ingredient amount of protein among the ingredients of target S, an ingredient amount of lipid, and an ingredient amount of carbohydrate. In addition, ingredient calculator 73 multiples the ingredient amount of protein, the ingredient amount of lipid, and the ingredient amount of carbohydrate by a calorie coefficient respectively corresponding to the ingredients, and sums these values so as to calculate a calorie of target S. Ingredient calculator 73 outputs results of them to display portion 80.

An example of display portion 80 is a liquid crystal screen. Display portion 80 has a function as a touch panel which is equipment capable of being selectively operated. In display portion 80, as an example, an analyzed result which is analyzed by analysis portion 70, a past analyzed result stored in memory portion 60, and the like are displayed.

Refer to FIG. 1 to FIG. 5, an action of food product analysis device 1 will be described.

As illustrated in FIG. 1, a user places target S on table 12, and starts analysis by food product analysis device 1. Irradiator 13 emits light to target S in response to start of the analysis by food product analysis device 1.

As illustrated in FIG. 2, the plurality of light receiving elements 41 receive light reflected from target S, and detect the light receiving amount. Light receiving element 41 outputs information relating to the absorbance of target S calculated on the basis of the detected light receiving amount to analysis portion 70 (refer to FIG. 3).

As illustrated in FIG. 3, analysis portion 70 calculates the first characteristic amount to the third characteristic amount from the absorbance of target S by characteristic amount calculator 71. In addition, analysis portion 70 matches the first characteristic amount to the third characteristic amount relating to target S and the first characteristic amount to the third characteristic amount relating to the sample by candidate determinator 72, and determines a plurality of the candidates by the matching. Analysis portion 70 outputs information relating to the candidates to display portion 80.

FIG. 4 is a front view illustrating a display example of a list of the candidates displayed on display portion 80 of food product analysis device 1 according to Embodiment 1 of the invention.

As illustrated in FIG. 4, display portion 80 displays information relating to candidates of the plurality of the food products. Display portion 80 displays a candidate A to a candidate J which are candidates of ten food products as the list of candidates. As an example, the candidate A which has the smallest matched result is the best matched food product, and the candidate J which has a tenth small matched result is a tenth matched food product. The candidate A to the candidate J indicate the types of the food products.

The user selects one food product from the candidates of the plurality of food products by operating display portion 80, which has a function of the touch panel, with a finger, or the like. The user selects a part where the candidate A are displayed by tapping with a finger, for example, in a case in which the food product corresponding to target S (refer to FIG. 1) is the candidate A.

As illustrated in FIG. 3, ingredient calculator 73 calculates the ingredient amount of protein, the ingredient amount of lipid, and the ingredient amount of carbohydrate among the ingredients of target S, and analysis portion 70 calculates calories from a result thereof. Analysis portion 70 (refer to FIG. 3) outputs the types of target S (refer to FIG. 1) which is the candidate A (refer to FIG. 4) and information relating to the ingredients of target S as an analyzed result to display portion 80.

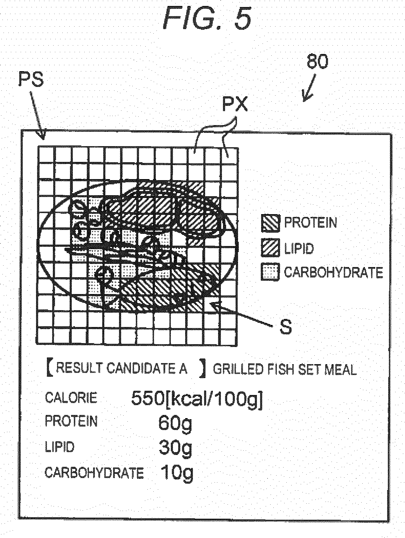

FIG. 5 is a front view illustrating a display example of the analyzed result displayed on display portion 80 of food product analysis device 1 according to Embodiment 1 of the invention.

As illustrated in FIG. 5, display portion 80 displays the types of target S, the information relating to the ingredients of target S, and an overlapped image PS in which a distribution image of ingredients included in target S and a photographic image of target S are overlapped as the analyzed result. FIG. 5 illustrates a direction of a line of hatching and a difference between display colors by dots.

The distribution image is, as an example, an image in which a positional relationship of the plurality of light receiving elements 41 (refer to FIG. 3) and the ingredients of target S calculated on the basis of light receiving element 41 are combined. The ingredient amounts of protein, lipid, and carbohydrate among the ingredients of target S are calculated in each region PX corresponding to a positional relationship of the plurality of light receiving elements 41 based on each of light receiving element 41. In the distribution image, an ingredient having the largest ingredient amount among the ingredients of target S is colored using a different display color according to the ingredient. The photographic image is, for example, an image which is generated on the basis of information by capturing target S using visible light (not illustrated). The overlapped image PS is generated by overlapping the distribution image and the photographic image.

Accordingly, the user can visually recognize the types of target S, the ingredient amount of target S, and the overlapped image PS relating to the ingredients of target S by displaying the analyzed result using food product analysis device 1.

Effects as follows can be obtained according to food product analysis device 1.
(1) Food product analysis device 1 analyzes the types of the food product by matching the first characteristic amount to the third characteristic amount relating to target S with the first characteristic amount to the third characteristic amount relating to the samples in the food product database. Since the first characteristic amount to the third characteristic amount is obtained on the basis of a result measured using the near infrared light, information other than target S is less likely to be included therein. Therefore, the first characteristic amount to the third characteristic amount relating to target S and the first characteristic amount to the third characteristic amount relating to the samples in the food product database are likely to be matched with each other. Therefore, food product analysis device 1 is capable of accurately analyzing the types of the food product.
   In addition, food product analysis device 1 calculates the ingredient amount of the ingredients of target S based on the absorbance of target S and the ingredient amount estimating model. Since the absorbance of target S is obtained on the basis of the result measured using the near infrared light, information other than target S is less likely to be included therein. Therefore, food product analysis device 1 is capable of accurately analyzing the ingredient amount of the food product.
(2) According to food product analysis device 1, information relating to protein, lipid, and carbohydrate, which are ingredients of the food product, are used for matching the first characteristic amount to the third characteristic amount relating to target S and the first characteristic amount to the third characteristic amount relating to the samples in the food product database with each other. Ingredients of these three food products are ingredients which are included a lot in the food product, and are ingredients which are likely to indicate characteristics of the food product. Therefore, ingredients of the three food products are used for matching, and thus accuracy of matching is improved. The types of the food product can be more accurately analyzed.
(3) As the matched result, the best matched the types of the food product and the types of the food product which is a target to be measured may be different from each other. According to food product analysis device 1, a plurality of results are output by matching, and a user selects one food product from the result. Accordingly, compared to a configuration in which the best matched one food product as a result of the matching is output, the food product which is a target to be measured is likely to be included in the result of the matching. Therefore, the types of the food product is likely to be accurately analyzed. Food product analysis device 1 has small targets to be selected, compared to a configuration of selecting the food products from the food product database. Therefore, it is less likely to take time and effort of the user.
(4) According to food product analysis device 1, the types of the food product and the ingredient amount of the food product are output as the analyzed result. In addition, since memory portion 60 stores the analyzed result, the user can easily look back the past analyzed result. Therefore, the user easily performs a nutritional management.

As described above, food product analysis device 1 of the embodiment is provided with memory portion 60 which stores the food product database containing multiple food product information. The food product information includes the characteristic information including at least one of the first light absorption information which relates to absorption of the near infrared light and the ingredient information which relates to the ingredients of the food product, and the type information which relates to the types of the food product. In addition, food product analysis device 1 is provided with analysis portion 70, and analysis portion 70 matches the measured information, which is the characteristic information obtained on the basis of the result measured using the near infrared light, with the information in the food product database.

Accordingly, food product analysis device 1 analyzes the types of the food product by matching the characteristic information in the food product database and the measured information with each other. Since the measured information is obtained on the basis of the result measured using the near infrared light, information other than the food product is less likely to be included in a relationship of the first light absorption information. Therefore, the characteristic information in the food product database and the measured information are likely to be matched with each other. Therefore, food product analysis device 1 is capable of accurately analyzing the types of the food product.

The plurality of results by the matching are determined, and the plurality of results may be output to a display equipment capable of being selectively operated. Accordingly, the plurality of results by the matching are output, and the user selects one food product from the result. Therefore, compared to a configuration in which the best matched one food product is output as a result of the matching, the food product which is a target to be measured is likely to be included in as the result of the matching. Therefore, the types of the food product is likely to be accurately analyzed.

Analysis portion 70 selects an estimation model based on the types of the food product determined by the matching, and may calculate any one of the ingredient amount and calories of the food product using the estimation model. Accordingly, at least one of the ingredient amount and the calories of the food product is likely to be accurately calculated by selecting the types of the food product or the estimation model corresponding to classification.

### (EMBODIMENT 2)

Food product analysis device 1 of Embodiment 2 is different from food product analysis device 1 of Embodiment 1 at a point to be described as follows, and has a substantially same configuration as that of food product analysis device 1 of Embodiment 1 at the other point. In description of food product analysis device 1 of Embodiment 2, same numerals are given to configurations thereof which are common to configurations of food product analysis device 1 of Embodiment 1, and a part or the entirety of the configurations are omitted.

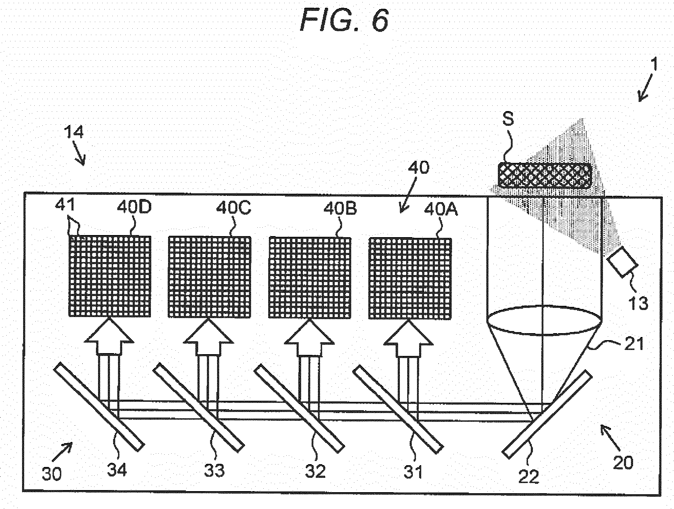

FIG. 6 is a schematic view illustrating a configuration of light receiver 14 of food product analysis device 1 according to Embodiment 2 of the invention.

As illustrated in FIG. 6, light dispersing portion 30 is provided with fourth light dispersing portion 34 in addition to first light dispersing portion 31, second light dispersing portion 32, and third light dispersing portion 33. Fourth light dispersing portion 34 is a spectroscope of a diffraction grating, which reflects only light of a specific wavelength and transmits light of the other wavelengths. Fourth light dispersing portion 34 selectively reflects light of wavelength relating to ingredients which are not included in the food product.

A plurality of light receiving sensors 40 includes fourth light receiving sensor 40D in addition to first light receiving sensor 40A, second light receiving sensor 40B, and third light receiving sensor 40C. Fourth light receiving sensor 40D receives the light reflected in fourth light dispersing portion 34. The plurality of light receiving elements 41 are disposed in a lattice shape on a light receiving surface of fourth light receiving sensor 40D.

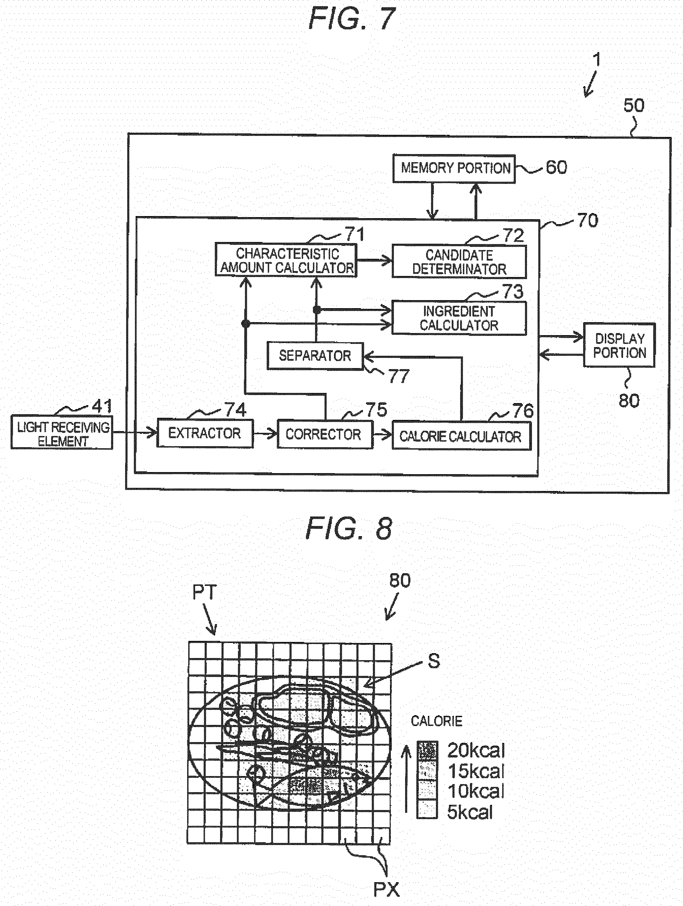

FIG. 7 is a block diagram of analysis unit 50 of food product analysis device 1 according to Embodiment 2 of the invention.

Refer to FIG. 7, a configuration of analysis unit 50 will be described.

Memory portion 60 stores an absorbance of the sample calculated on the basis of a light receiving amount detected by light receiving element 41, which is disposed on each of light receiving sensors 40A to 40C (refer to FIG. 6), and a calorie estimation model indicating a coefficient relationship with calories of the sample. In order to calculate the calorie estimation model, for example, a statistical technique of the multiple regression analysis, the PLS regression analysis, or the like is used. The calories of the samples are calculated in advance by another analysis method in the related art. In addition, memory portion 60 stores a reference absorbance, which is the absorbance of the sample calculated on the basis of the light receiving amount detected by light receiving element 41 disposed on fourth light receiving sensor 40D (refer to FIG. 6).

Analysis portion 70 of analysis unit 50 is provided with extractor 74, corrector 75, calorie calculator 76, and separator 77. Extractor 74 extracts target S (refer to FIG. 1) from a measuring range. Corrector 75 corrects the absorbance of target S. Calorie calculator 76 calculates calories of target S. Separator 77 separates target S and the others of target S from each other.

Extractor 74 calculates the absorbance of target S in each region where light receiving element 41 is disposed on the basis of the light receiving amount which is detected by light receiving element 41 disposed on each of light receiving sensors 40A to 40C. Extractor 74 extracts target S by detecting an edge of the calculated absorbance of target S based on a predetermined threshold. The predetermined threshold is, for example, a boundary whether the absorbance calculated from adjacent light receiving element 41 among the plurality of light receiving elements 41 has a constant difference or more. Extractor 74 determines that the large absorbance is target S, for example, in a case in which the absorbance calculated from adjacent light receiving element 41 among the plurality of light receiving elements 41 is equal to or more than a constant difference. Extractor 74 outputs information relating to the absorbance of target S to corrector 75 based on each of light receiving sensors 40A to 40C in the extracted target S.

Corrector 75 calculates the absorbance of target S based on the light receiving amount detected by light receiving element 41 disposed on fourth light receiving sensor 40D. Corrector 75 calculates influence information which is a difference between the calculated absorbance of target S and the reference absorbance. The influence information is, for example, an intensity of influence of an optical passage, a reflection rate of the food product, an environmental temperature, and the like. Corrector 75 corrects the absorbance of target S based on each of light receiving sensors 40A to 40C based on the influence information. Corrector 75 outputs information relating to the corrected absorbance of target S to characteristic amount calculator 71, ingredient calculator 73, and calorie calculator 76.

Calorie calculator 76 calculates calories of target S based on the corrected absorbance of target S and the calorie estimation model. Calorie calculator 76 outputs a calculated result to separator 77 and display portion 80.

Separator 77 separates target S and the others of target S from each other by separating a calculated result of the calories of target S based on the predetermined threshold. The predetermined threshold is, for example, a boundary of including or non-including calories. Separator 77 determines that the calories are included, for example, in a case in which the calculated result of the calories of target S is larger than the predetermined threshold. Separator 77 outputs information relating to the separation to characteristic amount calculator 71 and ingredient calculator 73.

Characteristic amount calculator 71 calculates the first characteristic amount to the third characteristic amount from the corrected absorbance of target S in a region of target S separated by separator 77. Ingredient calculator 73 calculates the ingredient amount of target S based on the corrected absorbance of target S and the ingredient amount estimating model in the region of target S separated by separator 77.

FIG. 8 is a front view illustrating a display example of the overlapped image relating to the calories displayed on display portion 80 of food product analysis device 1 according to Embodiment 2 of the invention.

As illustrated in FIG. 8, display portion 80 displays an overlapped image PT of which the distribution image of the calories of target S and the photographic image of target S are overlapped with each other. FIG. 8 illustrates a difference of display colors by a density of points of dots.

The distribution image is colored in each region PX using display colors which are different in response to the calories of target S. Accordingly, the user can visually recognize the overlapped image PT relating to the calories of target S displayed on display portion 80.

In addition, as illustrated in FIG. 5, display portion 80 displays the types of target S, information relating to the ingredients of target S, and an overlapped image PS in which the distribution image ingredients included in target S and the photographic image of target S are overlapped with each other as an analyzed result. Accordingly, the user can visually recognize the types of target S, the ingredient amount of target S, and, the overlapped image PS relating to the ingredients of target S by displaying the analyzed result using food product analysis device 1. It is preferable that food product analysis device 1 has, as an example, a configuration in which contents displayed in display portion 80 is switched by a switching button (not illustrated), or the like.

Food product analysis device 1 is capable of obtaining effects as follows, in addition to effects of (1) to (4) of Embodiment 1.
(5) Food product analysis device 1 extracts target S from the measuring range and calculates the absorbance of target S. Information other than target S is less likely to be included in the absorbance of target S. Therefore, the first characteristic amount to the third characteristic amount relating to target S and the first characteristic amount to the third characteristic amount relating to the samples of the food product database are further easily matched with each other. Therefore, the types of the food product can be more accurately analyzed.
(6) According to food product analysis device 1, the absorbance of target S based on light receiving sensor 40A to 40C is corrected on the basis of the influence information. Therefore, the absorbance of target S is more accurately calculated. Therefore, accuracy of the matching is further improved.
(7) According to food product analysis device 1, target S and the others of target S are separated from each other by separating a calculated result of the calories of target S based on the predetermined threshold. Therefore, when the characteristic amount is calculated from the absorbance of target S in the region of target S, information other than target S is less likely to be included in the characteristic amount. Therefore, accuracy of the matching is further improved.

As described above, food product analysis device 1 of the embodiment may calculate the measured information relating to the food product using a result in which the food product is extracted from the measuring range. Accordingly, the information other than the food product is less likely to be included in the measured information. Therefore, the characteristic information and the measured information of the food product database are further likely to be matched with each other. Therefore, the types of the food product can be more accurately analyzed.

In addition, ingredient information constituting the food product information may include information relating to an absolute amount of the ingredients of the food product. Accordingly, the matching of the food product database with the measured information are performed using the absolute amount of the ingredients of the food product. Therefore, the ingredient amount of the food product in conjunction with the types of the food product can be analyzed.

### (MODIFICATION EXAMPLE)

Specific embodiment which can be taken by the food product analysis device is not limited to the embodiments exemplified in each embodiment. The food product analysis device takes various embodiments which are different from each embodiment above described within a range where aims of the invention are achieved, within the scope of the appended claims. A modification example of each embodiment above described, which will be illustrated as follows, is an example of various embodiments which can be taken by the food product analysis device. Food product analysis device 1 of the modification example of Embodiment 1 includes a function of changing the measuring range. According to food product analysis device 1 of the modification example, the absorbance of target S is calculated on the basis of the light receiving amount detected from light receiving element 41 by analysis portion 70, and the absorbance thereof is converted to image data P relating to target S including information of brightness, color, or the like. Analysis portion 70 outputs image data P to display portion 80.

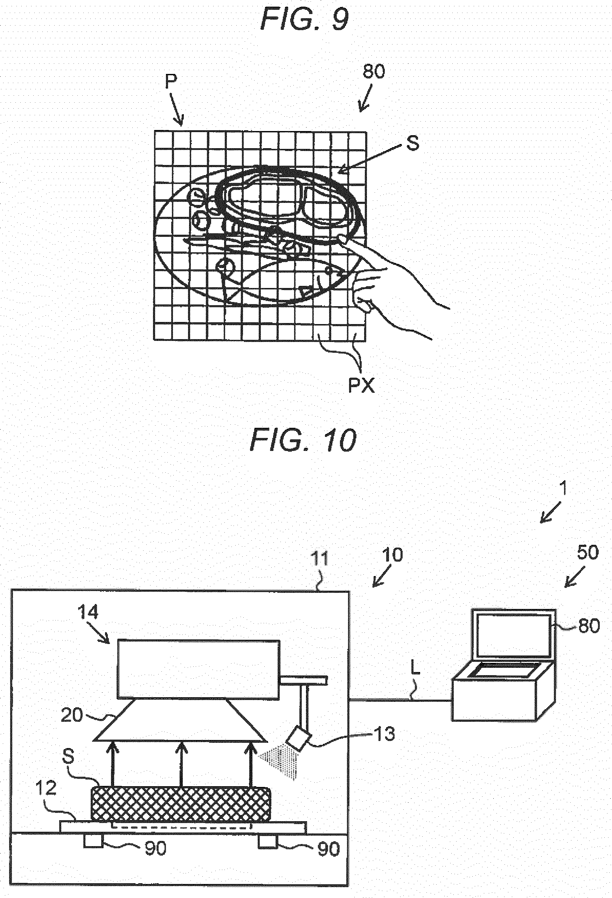

FIG. 9 is a front view illustrating a display example of the image data of the food product which is displayed on display portion 80 of food product analysis device 1 according to the modification example of the embodiment of the invention.

As illustrated in FIG. 9, display portion 80 displays image data P relating to target S. When the user selects a part of a range of which the food product is included in image data P, the measuring range is changed. Accordingly, food product analysis device 1 of the modification example starts an analysis of the measuring range of which a part of the food product of target S which is changed by the user is included.

Food product analysis device 1 of the modification example is capable of including, for example, a part of the food product of target S, by which a user wants to analyze, in the measuring range. Therefore, the information other than the food product is less likely to be included in the characteristic amount relating to the food product. Therefore, only the types of the food product, by which a user wants to analyze, can be accurately analyzed. Food product analysis device 1 of the modification example may have, for example, a configuration in which the others of the range selected by the user is set to the measuring range.
- Extractor 74 of the modification example of Embodiment 2 may extract target S using characteristics in which the absorption wavelength of a hydrogen bond is changed by a temperature. In this case, for example, another light dispersing portion which selectively reflects light of the absorption wavelength of water, and another light receiving sensor which receives light reflected in the light dispersing portion are included. According to the modification example, when target S is heated by microwave, or the like, target S is extracted on the basis of whether or not the absorption wavelength of water is peak-shifted.
- In food product analysis device 1 of the modification example of Embodiment 2, at least one of corrector 75, calorie calculator 76, and separator 77 may be omitted. For example, in food product analysis device 1 of the modification example, all of corrector 75, calorie calculator 76, and separator 77 may be omitted. According to the modification example, information relating to the absorbance of target S based on each of light receiving sensors 40A to 40C in target S extracted by extractor 74 is output to characteristic amount calculator 71 and ingredient calculator 73. In a case in which corrector 75 is omitted, fourth light dispersing portion 34 and fourth light receiving sensor 40D can be omitted.

FIG. 10 is a schematic view of food product analysis device 1 according to the modification example of the embodiment of the invention.
- Food product analysis device 1 of the modification example of each embodiment, as illustrated in FIG. 10, may be provided with pressure sensor 90 which measures a weight of target S, or the like disposed on table 12. In this case, memory portion 60 of food product analysis device 1 of the modification example stores, for example, information relating to a weight of a sample as one of another characteristic amount of the food product information. Therefore, according to food product analysis device 1 of the modification example, information relating to the weight of target S measured by pressure sensor 90 is matched with the information in the food product database as one of the characteristic amount. Therefore, the types of the food product are further accurately analyzed. In addition, according to the modification example, the calculated ingredients of target S may be corrected using the information relating to the weight of target S. Food product analysis device 1 of another modification example may be provided with a sensor (not illustrated), or the like which measures at least any one of a volume and an area of target S instead of, or in addition to pressure sensor 90.

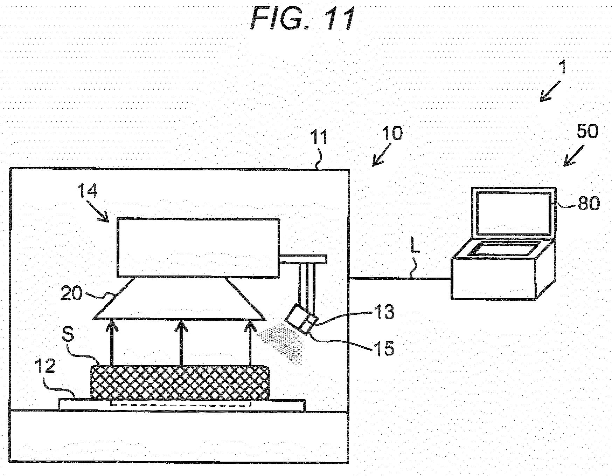

FIG. 11 is a schematic view of food product analysis device 1 according to the modification example of the embodiment of the invention.
- As illustrated in FIG. 11, food product analysis device 1 of the modification example of each embodiment may be provided with visible light irradiator 15 which applies visible light to target S. Visible light irradiator 15 is disposed on a position where light can be applied to the entirety of target S. In this case, memory portion 60 of food product analysis device 1 of the modification example stores, for example, a second light absorption information which relates to light-absorption of the visible light as one of another characteristic amount of the food product information. Therefore, according to food product analysis device 1 of the modification example, the characteristic amount relating to absorption of the visible light obtained when visible light irradiator 15 applies the visible light to target S, is matched with the food product database. Accordingly, for example, the types of fruit juice drinks in which ingredients are similar and colors are different can be analyzed in detail. Therefore, the types of the food product are further accurately analyzed.
- Food product analysis device 1 of the modification example of each embodiment may control an operation of the cooking appliances based on the types of the food product which are determined by the matching. According to food product analysis device 1 of the modification example, for example, heating time, power, and the like of a microwave oven, which is the cooking appliance, are controlled on the basis of the types of the food product which are determined by the matching. Therefore, a usability of the cooking appliances is improved. In food product analysis device 1 of another modification example, for example, a cooling temperature, and the like of a refrigerator which is the cooking appliance are controlled on the basis of the types of the food product which are determined by the matching.
- Food product analysis device 1 of the modification example of each embodiment selects another ingredient amount estimating model based on the types of the food product which are determined by the matching, and may calculate the ingredient amount of the food product using the other ingredient amount estimating model. In this case, memory portion 60 stores, for example, the other ingredient amount estimating model which is determined in each classification of the food products indicating a correlation between the absorbance of the sample calculated on the basis of the light receiving amount detected by light receiving element 41 and the ingredient amount of the sample. The other ingredient amount estimating model is calculated using, for example, the statistical method such as the multiple regression analysis or the PLS regression analysis. According to the modification example, the classification of target S, after the types of target S is analyzed, is determined by analysis portion 70, and ingredient calculator 73 calculates the ingredient amount of target S based on the absorbance of target S and the other ingredient amount estimating model. Therefore, when the other ingredient amount estimating model is selected in response to the determined classification of the food product, the ingredient amount of the food product is accurately calculated. The other ingredient amount estimating model may be determined in each of the types of the food products.

In a case of calculating calories, food product analysis device 1 of another modification example selects another calorie estimation model based on the types of the food product which are determined by the matching, and may calculate the calories of the food product using the calorie estimation model.
- In a case in which a plurality of the food products having the same types of the food product are analyzed, the characteristic amount of target S may be different due to a difference of the ingredient amount, or the like. Therefore, even when the types of the food product are the same as each other, the characteristic amount of target S and the characteristic amount of the food product information may be less likely to be matched with each other.

Food product analysis device 1 of the modification example of each embodiment may update the food product information based on the characteristic amount of target S which is used for the matching after the types of the food product is determined by the matching. According to the modification example, for example, an average of the characteristic amount of target S and the characteristic amount of the food product information which are matched with each other is stored as a characteristic amount of new food product information. In this case, the characteristic amount of the target and the characteristic amount of the food product information are likely to be matched with each other. Therefore, the types of the food product can be accurately analyzed. Food product analysis device 1 of the modification example may store an average of the characteristic amount of a plurality of targets S and the characteristic amount of the food product information which are matched as the characteristic amount of new food product information.
- Food product analysis device 1 of the modification example of each embodiment may store information relating to target S which are analyzed by a plurality of users through a network. For example, in a case in which the food product inexistent in the food product database is analyzed, the information of such as the types and the ingredient amount of the food product are stored in the food product database. Therefore, accuracy of the matching is improved by increasing an amount of information in the food product database. In addition, for example, the characteristic amount of the food product information corresponded on the basis of the matched characteristic amount of target S may be updated.
- Candidate determinator 72 of the modification example of each embodiment may determine 1% candidate of food product in the food product database in order of small one, for example, as a plurality of results determined by the matching. In addition, candidate determinator 72 of another modification example can arbitrary change N values which are determined through the matching by a user.
- In food product analysis device 1 of the modification example of each embodiment, candidate determinator 72 may be omitted. In this case, for example, one food product is determined by the matching.
- Memory portion 60 of the modification example of each embodiment may store food product database containing multiple food product information. The food product information includes the characteristic information including the ingredient information, which relates to the ingredients of a sample, and the type information which relates to the types of the sample. The ingredient information may be an each absolute value of the ingredient amount of protein, the ingredient amount of lipid, and the ingredient amount of carbohydrate. According to the modification example, characteristic amount calculator 71 calculates the ingredient amount of protein, the ingredient amount of lipid, and, the ingredient amount of carbohydrate of target S based on the absorbance of target S and the ingredient amount estimating model as the characteristic amount. Analysis portion 70 matches the calculated characteristic amount and the food product database with each other. Therefore, the ingredient amount of the food product in conjunction with the types of the food product can be analyzed. Therefore, a processing speed relating to analysis of food product analysis device 1 of the modification example is improved. In food product analysis device 1 of the modification example, ingredient calculator 73 can be omitted.
- Memory portion 60 of the modification example of each embodiment may store the food product database containing multiple food product information, which includes the characteristic information and the type information. The characteristic information includes the ingredient information, which relates to the ingredients of the sample, and the type information relates to the types of the sample. The ingredient information may be a configuration ratio of each ingredient of protein, lipid, and carbohydrate. In this case, memory portion 60 of the modification example further store a configuration ratio estimation model indicating a correlation of the absorbance of the sample calculated on the basis of the light receiving amount detected by light receiving element 41 and a configuration ratio of an ingredient of the sample. A statistical method such as the multiple regression analysis or the PLS regression analysis is used, for example, for calculating the configuration ratio estimation model. According to the modification example, characteristic amount calculator 71 calculates each configuration ratio of an ingredient protein, lipid, and carbohydrate of target S as the characteristic amount based on the absorbance and the configuration ratio estimation model of target S. Analysis portion 70 matches the calculated characteristic amount and the food product database with each other. Therefore, the configuration ratio of the ingredients of the food product hardly depends on volume and shape of the food product, and thus the calculated characteristic amount and the food product database are likely to be matched with each other. Therefore, the types of the food product can be accurately analyzed.
- Characteristic amount calculator 71 of the modification example of each embodiment calculates the absorbance of target S based on the light receiving amount which is detected from the plurality of light receiving elements 41 disposed on each of light receiving sensors 40A to 40C, and may calculates a sum of the absorbance as the first characteristic amount to the third characteristic amount relating to target S. In addition, characteristic amount calculator 71 of another modification example calculates a sum of the first characteristic amount to the third characteristic amount as the characteristic amount.
- Candidate determinator 72 of the modification example of each embodiment may match the first characteristic amount to the third characteristic amount with the first characteristic amount to the third characteristic amount relating to the samples in the food product database by weighting. In addition, candidate determinator 72 of another modification example sequentially matches the first characteristic amount to the third characteristic amount with the first characteristic amount to the third characteristic amount relating to the samples of the food product database in order from the highest characteristic amount.
- Candidate determinator 72 of the modification example of each embodiment matches each characteristic amount relating to target S and each characteristic amount relating to the sample using a primary function or a secondary function. In this case, coefficients of these functions may be calculated by weighting, for example. According to the modification example, a method such as full cross-validation or random validation may be used.
- In food product analysis device 1 of the modification example of each embodiment, ingredient calculator 73 may be omitted. In this case, for example, memory portion 60 stores the food product database containing the characteristic information including information, which relates to absorption of the near infrared light, and the food product information including information, which relates to the types, the ingredient amount, the calories and the like of the related food product. According to the modification example, the ingredient amount and the calories of the food product are output from the food product database.
- Food product analysis device 1 of the modification example of each embodiment may store a non-food product database containing multiple non-food product information including characteristic information, which includes at least one of the information relating to the absorption of the near infrared light and information relating to ingredients of the non-food product, and information relating to non-food products. According to the modification example, the food product and the others of the food product can be determined by the matching.
- Food product analysis device 1 of the modification example of each embodiment includes an operator (not illustrated). According to the invention the operator is a touch panel. For example, when the touch pad is operated by a user, a cursor (not illustrated) displayed on display portion 80 is operated, thereby making possible to selecting information displayed on display portion 80, or the like. According to the invention a portion 80.
- The plurality of light receiving elements 41, which are disposed in a circle, may be disposed in a concentric circle on a light receiving surface of light receiving sensor 40 of the modification example of each embodiment. In addition, when light receiving element 41 is disposed in a linear direction, and light receiving element 41 is moved in a direction orthogonal to the linear direction, light receiving element 41 can be also disposed simulatively in a two-dimension. Regarding a configuration in which light receiving element 41 is disposed in the linear direction, a light emitting time of irradiator 13 is changed in response to a moving time of light receiving element 41.
- One light receiving element 41 may be disposed on the light receiving surface of light receiving sensor 40 of the modification example of each embodiment. In this case, light receiving element 41 changes a region receiving light by a driving source (not illustrated), or the like. That is, light receiving element 41 receives light many times at one analysis.
- Light dispersing portion 30 of the modification example of each embodiment may be a spectroscope such as interference filter or prism. In addition, light dispersing portion 30 of another modification example is an acoustic optical element.
- Light dispersing portion 30 of the modification example of each embodiment may have a configuration in which only light of a specific wavelength is transmitted. According to the modification example, light receiving sensor 40 is disposed on a rear side of light dispersing portion 30.
- Food product analysis device 1 of the modification example of each embodiment may be provided with one, two, or four or more of light dispersing portion 30 and light receiving sensor 40. According to the modification example, instead of, or in addition to light dispersing portions 31 to 33 and light receiving sensors 40A to 40C, for example, another light dispersing portion which selectively reflects light of the absorption wavelength of water, and another light receiving sensor which receives the light reflected from the light dispersing portion are provided. Therefore, an amount of moisture, which is an ingredient included a lot in target S, and is an ingredient in which the characteristics of target S are easily appeared, can be analyzed. Therefore, accuracy of the matching is further improved. In addition, according to food product analysis device 1 of the modification example, wavelengths of specific light reflected by each of light dispersing portions 31 to 33 can be changed to wavelengths correlating to the calories of the food product, vitamins, or the like.
- In food product analysis device 1 of the modification example of each embodiment, one of irradiator 13 and light receiver 14 may be disposed on a lower side of target S. According to the modification example, when irradiator 13 emits the near infrared light to target S, light receiver 14 receives the light transmitted through target S.
- Irradiator 13 of the modification example of each embodiment may emit light having 700 nm to 1100 nm wavelength of the infrared region. A wavelength region of the infrared region can be changed arbitrary in a region including a wavelength correlating to the ingredient amount of the protein, lipid, and carbohydrate among the ingredients of the food product.
- Food product analysis device 1 of the modification example of each embodiment may use Raman spectroscopy which excites electrons of target S by outputting light of wavelengths other than the infrared region to target S. According to the modification example, after exciting the electrons of target S, irradiator 13 outputs the near infrared light.
- In food product analysis device 1 of the modification example of each embodiment, irradiator 13 may be omitted. In this case, for example, target S is analyzed using sunlight, or the near infrared light such as lighting of in interior which food product analysis device 1 is provided. According to the modification example, for example, target S is irradiated with the near infrared light by forming a slit which communicates with inside and outside of housing 11.

As described above, in Embodiment 1 and food product analysis device 1 of the modification example, the first light absorption information may include the information relating to the absorption of the near infrared light according to at least one ingredient among protein, lipid, carbohydrate, and moisture. In addition, the ingredient information may include information relating to at least one ingredient among protein, lipid, carbohydrate, and moisture.

Accordingly, the information relating to at least one ingredient among protein, lipid, carbohydrate, and moisture, which are the ingredients of the food product, is used for matching the characteristic information and the measured information of the food product database. These four ingredients of the food product is a ingredient which is included a lot in the food product, and is an ingredient in which the characteristics of the food product are easily appeared. Therefore, when these four ingredients of the food product are used for the matching, accuracy of the matching is improved. Therefore, the types of the food product can be more accurately analyzed.

The ingredient information constituting the food product information may include information relating to a configuration ratio of the ingredients of the food product. Accordingly, information relating to the configuration ratio of the ingredients of the food product is used for matching the characteristic information with the measured information of the food product database. The configuration ratio of the ingredients of the food product is less likely to depend on a volume and shape of the food product. Therefore, the volume and shape of the food product which is a target to be measured are less likely to affect on the matching. Therefore, the characteristic information and the measured information of the food product database are likely to be matched with each other. Therefore, the types of the food product can be accurately analyzed.

According to the invention, analysis portion 70 has a function of changing the measuring range as defined in appended claim 1. Accordingly, for example, only the food product by which the user wants to analyze is included in the measuring range, and thus the measured information relating to the food product is calculated. Therefore, the information other than the food product by which the user wants to analyze is less likely to be included in the measured information. Therefore, only the types of the food product, by which a user wants to analyze, can be accurately analyzed.

The food product information may further include the information relating to at least one of the weight, volume, and area of the food product. Accordingly, at least one information among the weight, volume, and area of the food product is added to the characteristic information including at least one of the first light absorption information and the ingredient information. Therefore, for example, the information can be narrowed down even more from a plurality of results obtained by matching the characteristic information including at least one of the first light absorption information and the ingredient information with the measured information, by matching the characteristic information including information of at least one of the weight, volume, and area of the food product with the measured information. Therefore, accuracy of the matching is likely to be further improved. Thus, the types of the food product is likely to be further accurately analyzed.

The food product information may further include second light absorption information which relates to absorption of the visible light. Accordingly, for example, the information can be narrowed down even more from the plurality of results obtained by matching the characteristic information including at least one of the first light absorption information and the ingredient information with the measured information, by matching the characteristic information including the second light absorption information and the measured information. Therefore, accuracy of the matching is likely to be further improved. Thus, the types of the food product are likely to be further accurately analyzed.

Analysis portion 70 may update the food product information based on the measured information used for matching after determining the types of the food product which are targets to be measured by the matching. Accordingly, the food product information is updated on the basis of one or a plurality of items of the measured information used for matching. For example, averages of the characteristic information in the food product database and one or the plurality of times of measured information used for matching are updated as characteristic information of new food product database. Therefore, in a case in which food products having the same types of the food product are analyzed again, the characteristic information in the food product database and the measured information are likely to be matched with each other. Therefore, the types of the food product are likely to be accurately analyzed.

Analysis portion 70 may control operation of the cooking appliances based on the types of the food product which are determined by the matching. Accordingly, facility of usage of the cooking appliances is improved.

### INDUSTRIAL APPLICABILITY

As described above, the food product analysis device according to an embodiment of the invention is useful for food product analysis device capable of accurately analyzing the types of the food product.

### REFERENCE MARKS IN THE DRAWINGS

- 1: food product analysis device
- 10: measuring unit
- 11: housing
- 12: table
- 13: irradiator
- 14: light receiver
- 15: visible light irradiator
- 20: light condenser
- 21: light condensing lens
- 22: reflector
- 30: light dispersing portion
- 31: first light dispersing portion
- 32: second light dispersing portion
- 33: third light dispersing portion
- 34: fourth light dispersing portion
- 40: light receiving sensor
- 40A: first light receiving sensor
- 40B: second light receiving sensor
- 40C: third light receiving sensor
- 40D: fourth light receiving sensor
- 41: light receiving element
- 50: analysis unit
- 60: memory portion
- 70: analysis portion
- 71: characteristic amount calculator
- 72: candidate determinator
- 73: ingredient calculator
- 74: extractor
- 75: corrector
- 76: calorie calculator
- 77: separator
- 80: display portion
- 90: pressure sensor

## Claims

1. A food product analysis device (1) comprising:
a memory portion (60) having stored therein a food product database containing a plurality of items of food product information including
characteristic information including at least one of
first light absorption information which is information relating to absorption of near infrared light and
ingredient information which is information relating to an ingredient of a food product, and
type information which is information relating to a type of the food product;
an analysis portion (70) configured to match measured information, which is the characteristic information obtained on the basis of a result of measurement using the near infrared light, with the food product database,
a display portion (80) in the form of a touch panel configured to display image data (P) showing a list of one or more candidates indicating the type of food product or an image of the food product and configured to allow a user to select one or more candidates or food product image parts by selecting a part of a range of the displayed image data (P);
wherein the analysis portion (70) has a function of extracting the measured information related to the selected one or more candidates or food product image parts according to said range selected by the user, and is further configured to process the measured information relating to the selected one or more candidates or food product image parts.

2. The food product analysis device (1) of claim 1,
wherein the first light absorption information includes information relating to absorption of the near infrared light of at least one ingredient of protein, lipid, carbohydrate, and water, and
wherein the ingredient information includes information relating to at least one ingredient of protein, lipid, carbohydrate, and water.

3. The food product analysis device (1) of claim 1,
wherein the analysis portion (70) is configured to determine a plurality of results in the matching, and to output the plurality of results to said display portion (80) in which a selection operation can be performed.

4. The food product analysis device (1) of claim 1,
wherein the ingredient information constituting the food product information includes information relating to a configuration ratio of the ingredients independent of a volume and shape of the food product.

5. The food product analysis device (1) of claim 1,
wherein the ingredient information constituting the food product information includes information relating to an absolute amount of the ingredients of the food product.

6. The food product analysis device (1) of claim 1,
wherein the food product information further includes information relating to at least one of a weight, a volume, and an area of the food product.

7. The food product analysis device (1) of claim 1,
wherein the food product information further includes second light absorption information which relates to absorption of visible light.

8. The food product analysis device (1) of claim 1,
wherein the analysis portion (70) is configured to update the food product information based on the measured information used for the matching after the analysis portion
(70) determines the type of the food product which is a target to be measured in the matching.

9. The food product analysis device (1) of claim 1,
wherein the memory portion (60) having stored therein an estimation model, and the analysis portion (70) is configured
to select the estimation model based on the type of the food product which is determined in the matching, and
to calculate at least one of an ingredient amount and calories of the food product using the estimation model.

10. The food product analysis device (1) of claim 1,
wherein the food product analysis device (1) is configured to control operation of a cooking appliance based on the type of the food product which is determined by the analysis portion (70) in the matching.

## Patentansprüche

1. Lebensmittel-Analysevorrichtung (1) umfassend:
einen Speicherabschnitt (60), auf dem eine Lebensmittel-Datenbank gespeichert ist, enthaltend eine Vielzahl von Elementen einer Lebensmittelinformation umfassend
eine charakteristische Information umfassend wenigstens ein Element der Gruppe umfassend
eine erste Lichtabsorptionsinformation, die eine Information in Bezug auf die Absorption von Nahinfrarotlicht ist, und
eine Zutateninformation, die eine Information in Bezug auf eine Zutat eines Lebensmittels ist, und
eine Typinformation, die eine Information in Bezug auf einen Typ des Lebensmittels ist;
einen zum Abgleichen der gemessenen Information, welche die auf der Basis eines Messergebnisses unter Verwendung des Nahinfrarotlichts erhaltene charakteristische Information ist, mit der Lebensmittel-Datenbank ausgebildeten Analyseabschnitt (70),
einen zum Anzeigen von Bilddaten (P) zur Darstellung einer Liste von einem oder mehreren Kandidaten zur Angabe des Typs von Lebensmittel oder eines Bildes des Lebensmittel ausgebildeten und zum Ermöglichen des Auswählens von einem oder mehreren Kandidaten oder Lebensmittel-Bildteilen durch Auswählen eines Teils eines Bereichs der angezeigten Bilddaten (P) für einen Benutzer ausgebildeten Anzeigeabschnitt (80) in der Form eines Touchscreens; wobei der Analyseabschnitt (70) eine Funktion zum Extrahieren der gemessenen Information in Bezug auf die ausgewählten einen oder mehreren Kandidaten oder Lebensmittel-Bildteile entsprechend dem vom Benutzer ausgewählten Bereich aufweist und ferner zum Verarbeiten der gemessenen Information in Bezug auf die ausgewählten einen oder mehreren Kandidaten oder Lebensmittel-Bildteile ausgebildet ist.

2. Lebensmittel-Analysevorrichtung (1) nach Anspruch 1,
wobei die erste Lichtabsorptionsinformation eine Information in Bezug auf die Absorption des Nahinfrarotlichts von wenigstens einer Zutat der Gruppe umfassend Eiweiß, Lipid, Kohlenhydrat und Wasser umfasst, und
wobei die Zutateninformation eine Information in Bezug auf wenigstens eine Zutat der Gruppe umfassend Eiweiß, Lipid, Kohlenhydrat und Wasser umfasst.

3. Lebensmittel-Analysevorrichtung (1) nach Anspruch 1,
wobei der Analyseabschnitt (70) ausgebildet ist zum Bestimmen einer Vielzahl von Ergebnissen im Abgleichen, und
zum Ausgeben der Vielzahl von Ergebnissen an den Anzeigeabschnitt (80), in dem eine Auswahloperation durchgeführt werden kann.

4. Lebensmittel-Analysevorrichtung (1) nach Anspruch 1,
wobei die die Lebensmittelinformation darstellende Zutateninformation eine Information in Bezug auf ein Konfigurationsverhältnis der Zutaten unabhängig von einem Volumen und einer Form des Lebensmittels umfasst.

5. Lebensmittel-Analysevorrichtung (1) nach Anspruch 1,
wobei die die Lebensmittelinformation darstellende Zutateninformation eine Information in Bezug auf eine absolute Menge der Zutaten des Lebensmittels umfasst.

6. Lebensmittel-Analysevorrichtung (1) nach Anspruch 1,
wobei die Lebensmittelinformation ferner eine Information in Bezug auf wenigstens ein Element der Gruppe umfassend ein Gewicht, ein Volumen und eine Fläche des Lebensmittels umfasst.

7. Lebensmittel-Analysevorrichtung (1) nach Anspruch 1,
wobei die Lebensmittelinformation ferner eine zweite Lichtabsorptionsinformation, die sich auf die Absorption von sichtbarem Licht bezieht, umfasst.

8. Lebensmittel-Analysevorrichtung (1) nach Anspruch 1, wobei der Analyseabschnitt (70) zum Aktualisieren der Lebensmittelinformation auf der Basis der zum Abgleichen verwendeten gemessenen Information ausgebildet ist, nachdem der Analyseabschnitt (70) den Typ des Lebensmittels bestimmt hat, der ein im Abgleichen zu messendes Ziel ist.

9. Lebensmittel-Analysevorrichtung (1) nach Anspruch 1,
wobei auf dem Speicherabschnitt (60) ein Schätzmodell gespeichert ist, und
der Analyseabschnitt (70) ausgebildet ist zum Auswählen des Schätzmodells auf der Basis des Typs des Lebensmittels, der im Abgleichen bestimmt wird, und
zum Berechnen einer Zutatenmenge oder/und von Kalorien des Lebensmittels unter Verwendung des Schätzmodells.

10. Lebensmittel-Analysevorrichtung (1) nach Anspruch 1,
wobei die Lebensmittel-Analysevorrichtung (1) zum Steuern des Betriebs eines Kochgeräts auf der Basis des Typs des Lebensmittels, der durch den Analyseabschnitt (70) im Abgleichen bestimmt wird, ausgebildet ist.

## Revendications

1. Dispositif d'analyse de produits alimentaires (1) comprenant :
une partie mémoire (60) comportant, stockée, une base de données de produits alimentaires contenant une pluralité d'éléments d'information sur des produits alimentaires incluant :
des informations caractéristiques incluant au moins un type parmi :
des informations de première absorption de lumière qui sont des informations se rapportant à l'absorption de la lumière dans le proche infrarouge, et
des informations d'ingrédient qui sont des informations se rapportant à un ingrédient du produit alimentaire, et
des informations de type qui sont des informations se rapportant au type de produits alimentaires,
une partie analyse (70) configurée pour faire correspondre des informations mesurées, qui représentent les informations caractéristiques obtenues sur la base d'un résultat de mesure en utilisant la lumière infrarouge avec la base de données de produits alimentaires,
une partie affichage (80) disponible sous la forme d'un panneau tactile configuré pour afficher des données d'image (P) représentant une liste d'un ou plusieurs candidats indiquant le type de produit alimentaire ou bien une image du produit alimentaire, et configuré pour permettre à un utilisateur de sélectionner un ou plusieurs candidats ou des parties d'images de produit alimentaire en sélectionnant une partie de la gamme des données d'image (P) affichées,
dans lequel la partie analyse (70) présente la fonction d'extraire les informations mesurées se rapportant à ce ou à ces candidats ou parties d'images de produit alimentaire sélectionnés en fonction de ladite gamme sélectionnée par l'utilisateur, et elle est en outre configurée pour traiter les informations mesurées se rapportant à ce ou à ces candidats ou parties d'images de produit alimentaire sélectionnés.

2. Dispositif d'analyse de produits alimentaires (1) selon la revendication 1,
dans lequel les informations de première absorption de lumière incluent des informations se rapportant à l'absorption de la lumière dans le proche infrarouge d'au moins un ingrédient parmi une protéine, un lipide, un glucide et de l'eau, et
dans lequel les informations d'ingrédient incluent des informations se rapportant à au moins un ingrédient parmi une protéine, un lipide, un glucide et de l'eau.

3. Dispositif d'analyse de produits alimentaires (1) selon la revendication 1,
dans lequel la partie analyse (70) est configurée pour déterminer une pluralité de résultats dans la mise en correspondance, et pour délivrer la pluralité de résultats à ladite partie affichage (80) dans laquelle une opération de sélection peut être effectuée.

4. Dispositif d'analyse de produits alimentaires (1) selon la revendication 1,
dans lequel les informations d'ingrédient constituant les informations sur les produits alimentaires incluent des informations se rapportant au taux de configuration des ingrédients indépendants du volume et de la forme du produit alimentaire.

5. Dispositif d'analyse de produits alimentaires (1) selon la revendication 1,
dans lequel les informations d'ingrédient constituant les informations sur les produits alimentaires incluent des informations se rapportant à la quantité en absolu des ingrédients du produit alimentaire.

6. Dispositif d'analyse de produits alimentaires (1) selon la revendication 1,
dans lequel les informations sur les produits alimentaires incluent en outre des informations se rapportant à l'un du poids, du volume et du domaine du produit alimentaire.

7. Dispositif d'analyse de produits alimentaires (1) selon la revendication 1,
dans lequel les informations sur les produits alimentaires incluent en outre des informations de seconde absorption de lumière qui se rapportent à l'absorption de la lumière visible.

8. Dispositif d'analyse de produits alimentaires (1) selon la revendication 1,
dans lequel la partie analyse (70) est configurée pour mettre à jour les informations sur les produits alimentaires sur la base des informations mesurées utilisées pour la mise en correspondance après que la partie analyse (70) a déterminé le type de produit alimentaire qui représente la cible à mesurer dans la mise en correspondance.

9. Dispositif d'analyse de produits alimentaires (1) selon la revendication 1,
dans lequel la partie mémoire (60) comporte un modèle d'estimation stocké en elle, et
la partie analyse (70) est configurée :
pour sélectionner le modèle d'estimation fondé sur le type de produit alimentaire déterminé lors de la mise en correspondance, et
pour calculer au moins l'un de la quantité d'ingrédients et des calories du produit alimentaire en utilisant le modèle d'estimation.

10. Dispositif d'analyse de produits alimentaires (1) selon la revendication 1,
le dispositif d'analyse de produit alimentaire (1) étant configuré pour piloter le fonctionnement d'un appareil de cuisson fondé sur le type de produit alimentaire qui est déterminé par la partie analyse (70) lors de la mise en correspondance.
